# EUROPÄISCHE PATENTANMELDUNG

(11) **EP 1 462 013 A1**
(43) Veröffentlichungstag der Anmeldung: **29.09.2004**
(21) Anmeldenummer: 03006986.8
(22) Anmeldetag: 27.03.2003
(51) Int. Cl.: A23P 1/04, C07C 37/00, C11B 5/00, A61K 47/48, A23L 1/30

(54) **Mikrokapseln, bestehend aus einer Hüllmembran und einer Hydroxytyrosol enthaltenden Matrix**

(71) Anmelder: Cognis Iberia, S.L., 08755 Castellbisbal, (Barcelona) (ES)
(72) Erfinder: Rull Prous, Santiago, 08034 Barcelona (ES); Fernandez-Blasquez, Jose, 08228 Terrassa (ES); Fabry, Bernd, Dr., 41352 Korschenbroich (DE)
(74) Vertreter: Fabry, Bernd, Dr.

(57) **Zusammenfassung**

Vorgeschlagen werden neue Mikrokapseln mit mittleren Durchmessern im Bereich von 0,0001 bis 5 mm, bestehend aus einer Hüllmembran und einer Hydroxytyrosol enthaltenden Matrix.

## Beschreibung

### Gebiet der Erfindung

Die Erfindung befindet sich auf dem Gebiet der biologischen Wirkstoffen und betrifft die Mikroverkapselung von Hydroxytyrosol, Verfahren zur Herstellung solcher Kapseln sowie deren Verwendung in verschiedenen Bereichen.

### Stand der Technik

Oleuropein stellt ein sehr wirksames natürliches Antioxidants dar, welches im wesentlichen aus den Blättern des Olivenbaums oder aus dem Abwasser der Olivenölherstellung gewonnen werden kann. Noch wirksamer ist jedoch Hydroxytyrosol, welches aus dem Oleuropein durch chemische oder enzymatische Spaltung gewonnen werden kann. Nachteilig jedoch ist, dass Hydroxytyrosol chemisch instabil ist und sowohl durch Sonnenlicht als auch durch den Einfluss von Luftsauerstoff sehr rasch oxidiert und damit unwirksam wird. Aus diesem Grunde ist die Bedeutung von Hydroxytyrosol als Wirkstoff für Kosmetik und Pharmazie bislang auch sehr gering, da die Halbewertszeit des Phenolderivats wesentlich kleiner als auch nur eine durchschnittliche Lagerzeit ist. Ein weiterer Nachteil besteht zudem darin, dass Hydroxytyrosol sich nur schwer in Formulierungen einarbeiten lässt und insbesondere mit vielen anderen typischen Bestandteilen unverträglich ist, d.h. in chemische Wechselwirkung tritt.

Die Aufgabe der vorliegenden Erfindung hat somit darin bestanden, Hydroxytyrosol in einer solchen Anbietungsform zur Verfügung zu stellen, dass die geschilderten Nachteile des Stands der Technik überwunden werden. Insbesondere sollte die (photo)chemische Beständigkeit des Wirkstoffes so gesteigert werden, dass nach einer Lagerung von 3 Monaten noch mindestens 90 % chemisch unverändert vorliegen. Außerdem sollte die neue Anbietungsform sicherstellen, dass der Wirkstoff auch mit einer unbegrenzt großen Zahl von kosmetischen oder pharmazeutischen Einsatzstoffen verwendet werden kann, ohne dass es zu chemischen Reaktionen zwischen den Komponenten kommt.

### Beschreibung der Erfindung

Gegenstand der Erfindung sind Mikrokapseln mit mittleren Durchmessern im Bereich von 0,0001 bis 5, vorzugsweise 0,001 bis 1 und insbesondere 0,01 bis 0,5 mm, bestehend aus einer Hüllmembran und einer Hydroxytyrosol enthaltenden Matrix. Im besonderen betrifft die Erfindung solche Mikrokapseln, die dadurch erhältlich sind, dass man
(a1) aus Gelbildnem, kationischen Polymeren Hydroxytyrosol sowie gegebenenfalls Polyalkylenglykolen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt,
oder
(b1) aus Gelbildnern, anionischen Polymeren, Hydroxytyrosol sowie gegebenenfalls Polyalkylenglykolen eine Matrix zubereitet,
(b2) gegebenenfalls die Matrix in einer Ölphase dispergiert,
(b3) die dispergierte Matrix mit wässrigen kationischer Polymere behandelt und gegebenenfalls dabei die Ölphase entfernt.
oder
(c1) eine wässrige Zubereitung von Hydroxytyrosol mit Ölkörpern sowie gegebenenfalls Polyalkylenglykolen in Gegenwart von Emulgatoren zu einer O/W-Emulsion verarbeitet,
(c2) die so erhaltene Emulsionen mit wässrigen Lösungen anionischer Polymere behandelt,
(c3) die so erhaltene Matrix mit wässrigen Lösungen kationischer Polymere in Kontakt bringt und
(c4) die so erhaltenen Verkapselungsprodukte von der wässrigen Phase abtrennt.

Überraschenderweise wurde gefunden, dass die Mikroverkapselung von Hydroxytyrosol dazu führt, dass das Produkt auch bei längerer Lagerung unter dem Einfluss von Luft und Sonnenlicht chemisch stabil bleibt. Die Kapseln lassen sich zudem problemlos in kosmetische oder pharamzeutische Zubereitungen, aber auch in Nahrungsmittelergänzungsmitteln formulieren, wobei das Problem der ansonsten häufig festzustellenden Unverträglichkeit mit anderen Wirkstoffen gleichzeitig mitgelöst wird. In einer bevorzugten Ausführungsform der vorliegenden Erfindung enthalten die Mikrokapseln in der Matrix, insbesondere aber in der Hülle kationische Polymere vom Typ des Chitosans. Die Erfindung schließt die Erkenntnis mit ein, dass Chitosane nicht nur wegen ihrer anti-inflammatorischen und schwach bakteriziden Wirkung wirksame Zusatzstoffe für kosmetische und pharmazeutische Zubereitungen darstellen, Hydroxytyrosin-Mikrokapseln mit einem Chitosangehalt erwiesen sich gegenüber Produkten auf Basis anderer kationischer Polymeren als abermals deutlich photostabiler.

### Gelbildner

Im Sinne der Erfindung werden als Gelbildner vorzugsweise solche Stoffe in Betracht gezogen, welche die Eigenschaft zeigen, in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin, solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Anionpolymere

Als anionische Polymere eignen sich neben anionischen Polysacchariden, wie z.B. Carboxymethylcellulose, oder Poly(meth)acrylsäuren und deren Derivaten, wie z.B. Salzen und Estern, vorzugsweise Salze der Alginsäure. Bei der Alginsäure handelt es sich um ein Gemisch carboxylgruppenhaltiger Polysaccharide mit folgendem idealisierten Monomerbaustein:

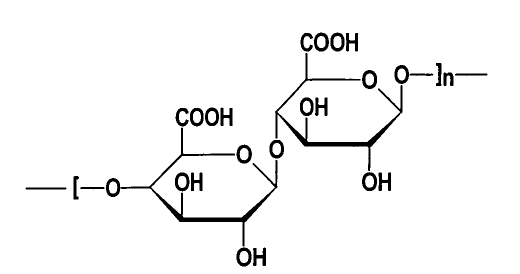

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Die Einsatzmenge der Anionpolymere beträgt in der Regel - bezogen auf die Mikrokapseln - 0,01 bis 1, vorzugsweise 0,05 bis 0,1 Gew.-%.

### Polyalkylenglykole

Der Zusatz der Polyalkylenglykole führt zu einer Stabilisierung der Kapseln und verbessert insbesondere ihre Resistenz gegen den Einfluss von Tensiden. Bei diesen Polymeren kann es sich um Oligomere des Ethylenoxids oder Propylenoxids bzw. um Gemische dieser beiden - in Random- wie in Blockverteilung - handeln. Üblicherweise kommen solche Polyalkylenglykole, vorzugsweise aber Polyethylenglykole zum Einsatz, die ein mittleres Molekulargewicht von 100 bis 5.000, vorzugsweise von 200 bis 2.000 aufweisen.

### Ölphase

In einer besonderen Ausführungsform der Erfindung kann die im ersten Schritt hergestellte Matrix vor der Verkapselung in einer Ölphase dispergiert werden. Für diesen Zwecke kommen beispielsweise in Frage : Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetyhnyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₅-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane. Die Menge der Ölkörper kann bezogen auf die Mikrokapseln 10 bis 30 und vorzugsweise 15 bis 30 Gew.-% betragen.

### Emulgatoren

Für den Fall, dass die Matrix vor der Verkapselung in einer Ölphase dispergiert wird, empfiehlt es sich, Emulgatoren mitzuverwenden, um eine homogene Dispersion herzustellen. Als Emulgatoren kommen grundsätzlich anionische, kationische oder ampholytische Tenside in Frage. Vorzugsweise werden jedoch nichtionogene Tenside eingesetzt, welche beispielsweise aus mindestens einer der folgenden Gruppen ausgewählt sein können.
- Anlagerungsprodukte von 2 bis 30 Mol Ethylenoxid und/ oder 0 bis 5 Mol Propylenoxid an lineare Fettalkohole mit 8 bis 22 Kohlenstoffatomen, an Fettsäuren mit 12 bis 22 Kohlenstoffatomen, an Alkylphenole mit 8 bis 15 Kohlenstoffatomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Die Konzentration der Emulgatoren kann bezogen auf die Mikrokapseln 1 bis 10 und vorzugsweise 2 bis 8 Gew.-% betragen.

### Kationpolymere

Typische Beispiele sind beispielsweise Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, Kondensationsprodukte aus Dihalogenalkylen, wie z.B. Dibrombutan mit Bisdialkylaminen, wie z.B. Bis-Dimethylamino-1,3-propan, sowie quatemierte Ammoniumsalz-Polymere, wie z.B. Mirapol® A-15, Mirapol® AD-1, Mirapol® AZ-1 der Firma Miranol. Die Einsatzmenge der Kationpolymere liegt - bezogen auf die Mikrokapseln - vorzugsweise im Bereich von 0,01 bis 1, vorzugsweise 0,05 bis 0,1 Gew.-%.

Wie schon erläutert, werden in einer bevorzugten Ausführungsform der Erfindung kationische Biopolymere vom Typ der Chitosane eingesetzt. Chitosane werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

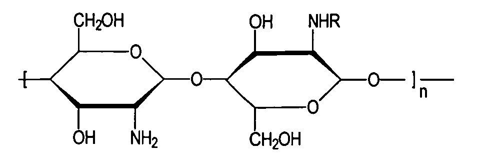

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Herstellung der Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Anion- bzw. das Kationpolymer in Mengen von 0,1 bis 2, vorzugsweise 0,25 bis 0,5 Gew.-%, das Hydroxytyrosol in Mengen von 0,1 bis 25 und insbesondere 0,25 bis 10 Gew.-% sowie den Polyalkylenglykol in Mengen von 0,1 bis 5 und insbesondere 1 bis 2 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Polymer und Hydroxytyrosol kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C gatorischen Schritt erfolgt dann die eigentliche Verkapselung, d.h. die Ausbildung der Hüllmembran durch Inkontaktbringen des Polymers in der Matrix mit den entgegengesetzt geladenen anderen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des anderen Polymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,1 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

In einem alternativen Verfahren wird zur Herstellung der erfindungsgemäßen Mikrokapseln wird zunächst eine O/W-Emulsion zubereitet, welche neben dem Ölkörper, Wasser, dem Hydroxytyrosol und gegebenenfalls den Polyalkylenglykolen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Kationpolymerlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

### Gewerbliche Anwendbarkeit

Ein weiterer Gegenstand der Erfindung betrifft die Verwendung der erfindungsgemäßen Mikrokapseln zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben. Diese können ihrerseits wieder Wirkstoffe, Ölkörper und Emulgatoren in unverkapselter Form enthalten, wie sie bereits eingangs beschrieben worden sind; auf eine erneute Aufzählung wird daher verzichtet. Bei den Zubereitungen kann es sich auch um Lebensmittelzusatzstoffe handeln, beispielsweise um Additive für Sportlernahrung und dergleichen. Die Mikrokapseln können in diesen Mitteln in Mengen von 1 bis 50, vorzugsweise 5 bis 15 Gew.-% enthalten sein.

### Beispiele

### Beispiel 1

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt. Ein Muster der Mikrokapseln und eine Refernzprobe unverkapselten Hydroxytyrosins wurden 4 Wochen bei 20 °C in einem verschlossenen Gefäß im Sonnenlicht gelagert. Während der Hydroxytyrosingehalt der Referenzprobe nach der Lagerung noch 26 % betrug, lag der Gehalt in der erfindungsgemäßen Probe bei 96 %.

### Beispiel 2

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit 20 g einer 1 Gew.-%igen Lösung von Chitosan in Glycolsäure, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 10 Gew.-%ige wässrige Lösung von Natriumalginat getropft. Zum Erhalt von Mikrokapseln gleichen Durchmessers wurden die Zubereitungen anschließend gesiebt. Während der Hydroxytyrosingehalt der Referenzprobe nach der Lagerung noch 24 % betrug, lag der Gehalt in der erfindungsgemäßen Probe bei 97 %.

### Beispiel 3

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige wässrige Lösung von Polyquart® Ampho 149 (Cognis) getropft. Zum Erhalt von Mikrokapseln gleichen Durchmessers wurden die Zubereitungen anschließend gesiebt. Während der Hydroxytyrosingehalt der Referenzprobe nach der Lagerung noch 26 % betrug, lag der Gehalt in der erfindungsgemäßen Probe bei 75 %.

### Beispiel 4

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige wässrige Lösung von Merquat® 550 (Chemviron) getropft. Zum Erhalt von Mikrokapseln gleichen Durchmessers wurden die Zubereitungen anschließend gesiebt. Während der Hydroxytyrosingehalt der Referenzprobe nach der Lagerung noch 25 % betrug, lag der Gehalt in der erfindungsgemäßen Probe bei 78 %.

### Beispiel 5

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige wässrige Lösung von Mirapol® A15 (Miranol) getropft. Zum Erhalt von Mikrokapseln gleichen Durchmessers wurden die Zubereitungen anschließend gesiebt. Während der Hydroxytyrosingehalt der Referenzprobe nach der Lagerung noch 27 % betrug, lag der Gehalt in der erfindungsgemäßen Probe bei 73 %.

### Beispiel 6

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Hydroxytyrosol, 1 g PEG-200, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige wässrige Lösung von Amodimethicone getropft. Zum Erhalt von Mikrokapseln gleichen Durchmessers wurden die Zubereitungen anschließend gesiebt. Während der Hydroxytyrosingehalt der Referenzprobe nach der Lagerung noch 24 % betrug, lag der Gehalt in der erfindungsgemäßen Probe bei 67 %.

## Patentansprüche

1. Mikrokapseln mit mittleren Durchmessern im Bereich von 0,0001 bis 5 mm, bestehend aus einer Hüllmembran und einer Hydroxytyrosol enthaltenden Matrix.

2. Mikrokapseln nach Anspruch 1, dadurch erhältlich, dass man
(a1) aus Gelbildnern, kationischen Polymeren Hydroxytyrosol sowie gegebenenfalls Polyalkylenglykolen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.

3. Mikrokapseln nach Anspruch 1, dadurch erhältlich, dass man
(b1) aus Gelbildnern, anionischen Polymeren, Hydroxytyrosol sowie gegebenenfalls Polyalkylenglykolen eine Matrix zubereitet,
(b2) gegebenenfalls die Matrix in einer Ölphase dispergiert,
(b3) die dispergierte Matrix mit wässrigen kationischer Polymere behandelt und gegebenenfalls dabei die Ölphase entfernt.

4. Mikrokapseln nach Anspruch 1, dadurch erhältlich, dass man
(c1) eine wässrige Zubereitung von Hydroxytyrosol mit Ölkörpern sowie gegebenenfalls Polyalkylenglykolen in Gegenwart von Emulgatoren zu einer O/W-Emulsion verarbeitet,
(c2) die so erhaltene Emulsionen mit wässrigen Lösungen anionischer Polymere behandelt,
(c3) die so erhaltene Matrix mit wässrigen Lösungen kationischer Polymere in Kontakt bringt und
(c4) die so erhaltenen Verkapselungsprodukte von der wässrigen Phase abtrennt.

5. Verfahren zur Herstellung von Mikrokapseln mit mittleren Durchmessern im Bereich von 0,0001 bis 5 mm, bestehend aus einer Hüllmembran und einer Hydroxytyrosol enthaltenden Matrix, bei dem man
(a1) aus Gelbildnern, kationischen Polymeren und Hydroxytyrosol eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.

6. Verfahren zur Herstellung von Mikrokapseln mit mittleren Durchmessern im Bereich von 0,0001 bis 5 mm, bestehend aus einer Hüllmembran und einer Hydroxytyrosol enthaltenden Matrix, bei dem man
(b1) aus Gelbildnern, anionischen Polymeren und Hydroxytyrosol eine Matrix zubereitet,
(b2) gegebenenfalls die Matrix in einer Ölphase dispergiert,
(b3) die dispergierte Matrix mit wässrigen kationischer Polymere behandelt und gegebenenfalls dabei die Ölphase entfernt.

7. Verfahren zur Herstellung von Mikrokapseln mit mittleren Durchmessern im Bereich von 0,0001 bis 5 mm, bestehend aus einer Hüllmembran und einer Hydroxytyrosol enthaltenden Matrix, bei dem man
((c1) eine wässrige Zubereitung von Hydroxytyrosol mit Ölkörpern in Gegenwart von Emulgatoren zu einer O/W-Emulsion verarbeitet,
(c2) die so erhaltene Emulsionen mit wässrigen Lösungen anionischer Polymere behandelt,
(c3) die so erhaltene Matrix mit wässrigen Lösungen kationischer Polymere in Kontakt bringt und
(c4) die so erhaltenen Verkapselungsprodukte von der wässrigen Phase abtrennt.

8. Verfahren nach mindestens einem der Ansprüche 5 bis 7 **dadurch gekennzeichnet, dass** man Gelbildner einsetzt, die ausgewählt sind aus der Gruppe, die von Heteropolysacchariden und Proteinen gebildet wird.

9. Verfahren nach mindestens einem der Ansprüche 5 bis 8, **dadurch gekennzeichnet, dass** man fakultativ Polyalkylenglykole einsetzt, die ein mittleres Molekulargewicht im Bereich von 100 bis 5.000 aufweisen.

10. Verfahren nach mindestens einem der Ansprüche 5 bis 9, **dadurch gekennzeichnet, dass** man anionische Polymere einsetzt, die ausgewählt sind aus der Gruppe, die gebildet wird von anionischen Polysacchariden, Poly(meth)acrylsäuren und deren Derivaten sowie Alginsäure und deren Salzen.

11. Verfahren nach mindestens einem der Ansprüche 5 bis 10, **dadurch gekennzeichnet, dass** man die Matrix in einer Ölphase dispergiert, die gebildet wird von Guerbetalkoholen auf Basis von Fettalkoholen mit 6 bis 18 Kohlenstoffatomen; Estern von linearen C₆-C₂₂-Fettsäuren mit linearen oder verzweigten C₆-C₂₂-Fettalkoholen; Estern von verzweigten C₆-C₁₃-Carbonsäuren mit linearen oder verzweigten C₆-C₂₂-Fettalkoholen; Estern von linearen C₆-C₂₂-Fettsäuren mit verzweigten Alkoholen; Estern von C₁₈-C₃₈-Alkylhydroxycarbonsäuren mit linearen oder verzweigten C₆-C₂₂-Fettalkoholen; Estern von linearen und/oder verzweigten Fettsäuren mit mehrwertigen Alkoholen und/oder Guerbetalkoholen; Triglyceriden auf Basis C₆-C₁₀-Fettsäuren; flüssigen Mono-/Di/Triglyceridmischungen auf Basis von C₆-C₁₈-Fettsäuren; Estern von C₆-C₂₂-Fettalkoholen und/oder Guerbetalkoholen mit aromatischen Carbonsäuren; Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen; pflanzlichen Ölen; verzweigten primären Alkoholen; substituierten Cyclohexanen; linearen und verzweigte C₆-C₂₂-Fettalkoholcarbonaten; Guerbetcarbonaten auf Basis von Fettalkoholen mit 6 bis 18 Kohlenstoffatomen; Estern der Benzoesäure mit linearen und/oder verzweigten C₆-C₂₂-Alkoholen; linearen oder verzweigten, symmetrischen oder unsymmetrischen Dialkylethern mit 6 bis 22 Kohlenstoffatomen pro Alkylgruppe; Ringöffnungsprodukten von epoxidierten Fettsäureestern mit Polyolen; Siliconölen sowie aliphatischen bzw. naphthenischen Kohlenwasserstoffen und Mineralölen sowie deren Gemischen.

12. Verfahren nach mindestens einem der Ansprüche 5 bis 11, **dadurch gekennzeichnet, dass** man synthetische Kationpolymere einsetzt, die ausgewählt sind aus der Gruppe, die gebildet wird von Copolymeren von Diallylammoniumsalzen und Acrylamiden, quaternierten Vinylpyrrolidon/Vinylimidazol-Polymeren, Kondensationsprodukten von Polyglycolen und Aminen, Polyethyleniminen, kationischen Siliconpolymeren, Copolymeren der Adipinsäure und Dimethylaminohydroxypropyldiethylentriamin, Copolymeren der Acrylsäure mit Dimethyldiallylammoniumchlorid, Polyaminopolyamiden sowie deren vernetzten wasserlöslichen Polymeren, Kondensationsprodukten aus Dihalogenalkylen und Bisdialkylaminen sowie quaternierten Ammoniumsalzpolymeren.

13. Verfahren nach mindestens einem der Ansprüche 5 bis 12, **dadurch gekennzeichnet, dass** man natürliche Kationpolymere vom Typ des Chitosans einsetzt.

14. Verwendung von Mikrokapseln nach Anspruch 1 zur Herstellung kosmetischer oder pharmazeutischer Zubereitungen.

15. Verwendung von Mikrokapseln nach Anspruch 1 als Nahrungsmittelergänzungsstoffe.
